# EUROPÄISCHE PATENTSCHRIFT

(11) **EP 1 093 524 B1**
(45) Veröffentlichungstag und Bekanntmachung des Hinweises auf die Patenterteilung: **16.02.2005**
(21) Anmeldenummer: 98945301.4
(22) Anmeldetag: 01.09.1998
(51) Int. Cl.: C12N 15/86, C12N 7/01, C12N 7/04, C12N 5/10

(54) **REKOMBINANTE HERPESVIREN FÜR DIE ERZEUGUNG REKOMBINANTER ADENO-ASSOZIIERTER-VIREN**
RECOMBINANT HERPES VIRUSES FOR PREPARING RECOMBINANT ADENO-ASSOCIATED VIRUSES
VIRUS HERPETIQUES RECOMBINES POUR LA PRODUCTION DE VIRUS RECOMBINES ASSOCIES AUX ADENOVIRUS

(30) Priorität: 06.07.1998 DE 19830141
(43) Veröffentlichungstag der Anmeldung: 25.04.2001
(73) Patentinhaber: APPLIED GENETIC TECHNOLOGIES CORPORATION, Alachua, FL 32615 (US)
(72) Erfinder: HEILBRONN, Regine, D-12203 Berlin (DE); SCHETTER, Christian, D-40723 Hilden (DE)
(74) Vertreter: Weiss, Wolfgang, Dipl.-Chem. Dr.
(86) Internationale Anmeldenummer: PCT/EP1998/005542
(87) Internationale Veröffentlichungsnummer: WO 2000/001834

(56) Entgegenhaltungen:
- WO-A-95/06743
- WO-A-95/20671
- RIXON F J AND MCLAUCHLAN: "Insertion of DNA sequences at a unique restriction enzyme site engineered for vector purposes into the genome of herpes simplex virus type 1" JOURNAL OF GENERAL VIROLOGY, Bd. 113, Nr. 71, 1. Januar 1990, Seite 2931 2939 XP002079295 in der Anmeldung erwähnt
- SRIVASTAVA A ET AL: "NUCLEOTIDE SEQUENCE AND ORGANIZATION OF THE ADENO-ASSOCIATED VIRUS 2 GENOME" JOURNAL OF VIROLOGY, Bd. 45, Nr. 2, 1. Februar 1983, Seiten 555-564, XP002058633 in der Anmeldung erwähnt
- CONWAY ET AL.: "Recombinant adeno-associated virus type 2 replication and packaging is entirely supported by herpes simplex virus type 1 amplicon expressing rep and cap" JOURNAL OF VIROLOGY, Bd. 71, Nr. 11, November 1997, Seiten 8780-8789, XP002102271 in der Anmeldung erwähnt

## Beschreibung

Die Erfindung betrifft einen rekombinanten Herpesvirus, ein Verfahren zur dessen Herstellung sowie ein Verfahren zur Herstellung von hochtitrigen, infektiösen Adeno-assoziierten Virus-Vektor-Präparationen.

Es sind zahlreiche genetische Erkrankungen bekannt, für die bisher keine wirksame Therapie gefunden wurde. Ein möglicher Weg zur Behandlung genetischer Erkrankungen besteht darin, ein bestimmtes Gen, welches die genetische Erkrankung korrigieren kann, in das Genom des Patienten einzubringen, so daß es in der DNA des Patienten vorliegt und sich dort replizieren kann.

Zum Einbringen des gewünschten korrigierenden Gens in die Zelle können Vektoren verwendet werden, wobei virale Vektoren auf der Basis von Adeno-assoziierten Viren (AAV) für die Gentherapie gegenüber anderen viralen Vektorsystemen eine Reihe von Vorteilen aufweisen. AAV ist ein sehr stabiles, aber apathogenes Virus. Zudem hat es einen breiten Gewebetropismus und die Fähigkeit zur effizienten chromosalen Integrationen sowohl in proliferierenden als auch in ruhenden Zellen.

Als virale Vektorsysteme werden zumeist AAV-Vektoren auf Basis des Adeno-assoziierten Virus Typ 2 (AAV-2) eingesetzt (Carter, Curr. Opinion. Biotech. 3 (1992) 533; Muzyczka, Curr. Top. Microbiol. Imm. 158 (1992) 97). Bei AAV-2 handelt es sich um ein weit verbreitetes, humanes Virus, wobei bei der Primärinfektion, die zumeist im Kindesalter stattfindet, keine Pathogenität induziert wird. Es gibt auch keine Hinweise darauf, daß AAV-2 ein onkogenes Potential besitzen könnte. Aufgrund der hohen Stabilität von AAV-2 kann das Virus auch aufwendige Reinigungsverfahren ohne Verlust an Infektiösität überstehen.

AAV-2 ist ein kleines, einzelsträngiges DNA-Virus, das zwei Gene trägt: Das rep-Gen kodiert für vier überlappende, regulatorische Proteine, Rep78 und Rep52 und C-terminal gepleißte Varianten der beiden Proteine, Rep68 und Rep40. Die Rep-Proteine dienen zur AAV-Genregulation, DNA-Replikation und Virusverpackung. Das cap-Gen kodiert für die drei Virus-Capsidproteine. Die beiden Gene rep und cap werden von 145 bp langen invertierten Repititionen flankiert, die als "Origins of Replication" dienen. Diese sind die einzigen AAV DNA-Sequenzen, die in Cis für DNA-Replikation, Virusverpackung und Integration in Wirtszellgenom benötigt werden, so daß ca. 4,7 kb lange Fremdsequenzen in die jeweiligen Vektoren kloniert werden können.

AAV sind replikationsdefekt und benötigen für eine effiziente Vermehrung eine Coinfektion mit einem Helfervirus. Ohne Helferviren integriert AAV mit hoher Frequenz ins Wirtszellgenom, wobei ein spezifischer Lokus auf Chromosom 19 (q13.3-qter) stark bevorzugt wird. Die Integration ist dabei unabhängig von der viralen oder zellulären DNA-Replikation. Aufgrund der genannten Vorzüge bieten AAV-Vektoren ideale Voraussetzungen für die Einschleusung und stabile Integration von Genen in nicht prolifierierende Zellen. Für die Gentherapie werden üblicherweise rekombinante AAV-Vektoren eingesetzt, die eine heterologe, für ein gewünschtes Genprodukt kodierende DNA umfassen. Durch die Insertion der heterologen DNA in die AAV-Vektoren wird jedoch die rep und cap-Expression beeinträchtigt. Deshalb müssen zur Vermehrung der rekombinanten AAV-Vektoren diese Proteine bzw. Systeme, die diese Proteine exprimieren, von außen zugeführt werden.

Diese Vermehrung kann beispielsweise durch Cotransfektion von Adenovirus-infizierten Zellen mit dem rekombinanten AAV-Vektor und einem Helferplasmid erfolgen, im dem die rep- und cap-Gene von AAV durch die Replikationsursprünge ("Origins of Replication") von Adenovirus Typ 5 flankiert werden. Das Helferplasmid wird in den Adenovirus-infizierten Zellen repliziert, so daß ausreichende Mengen an Rep und Cap zur Propagation des AAV-Vektors zur Verfügung stehen. Das rep- und cap-exprimierende Konstrukt weist keine gemeinsamen DNA-Sequenzen mit dem AAV-Vektor auf, so daß kein die Vektorpräparation kontaminierendes Wildtyp AAV durch homologe Rekombination entstehen kann (Samulski et al., J. Virol. 63 (1989), 3822). Ein Nachteil dieses Systems ist jedoch, daß die erreichbaren Virustiter mit 10⁴ bis 10⁶ infektiösen Partikeln pro ml deutlich niedriger als bei Wildtyp-AAV sind, das bis zu 10¹² Partikel pro ml erreichen kann. Zudem müssen für jeden Ansatz aufs neue Zellen mit dem AAV-Vektor und dem rep- und cap-exprimierenden Helferplasmid cotransfiziert und anschließend mit Adenovirus infiziert werden, da das Helferplasmid nicht als Virus verpackt wird und somit nicht durch Infektion übertragen werden kann.

J. Conway et al. (J. Virol. 71 (11) (1997), 8780-8789) beschreiben ein Verfahren zur Replikation und Verpackung von rekombinantem AAV Typ 2 mittels eines Herpes Simplex Virus (HSV) Typ 1 Amplikons, das Rep und Cap exprimiert. Die HSV-Amplikons werden in HSV-Hüllen verpackt und können deshalb nur in Gegenwart von Wildtyp-HSV vermehrt werden. Das relative Verhältnis von Wildtyp HSV und verpackten Amplikons ist bei der gemeinsamen Vermehrung nicht vorhersehbar und auch kaum einstellbar. Es gibt auch keine Methode, die beiden Viruspopulationen durch Aufreinigung voneinander zu trennen, da die beiden gemeinsame Hülle die wesentlichen physiko-chemischen Eigenschaften ausmacht. Darüber hinaus besteht das Problem, daß das Amplikon aufgrund von Wachstumsnachteilen bereits nach wenigen Passagen nur noch in einer nicht mehr nachweisbar geringen Menge vorliegt. Das Amplikon-System ist deshalb nur reproduzierbar anwendbar, wenn man für jede rAAV-Präparation das Amplikon-Plasmid transfiziert und dann mit Wildtyp-HSV überinfiziert. Dies hat aber zur Folge, daß man für jede Vermehrungsrunde die Amplikon-Plasmide erneut transfizieren muß, was aber genau der Nachteil des weiter oben beschriebenen Verpackungssystems ist.

Es wurde versucht, die mit der Verwendung von nicht selbstreplizierenden Plasmiden verbundene Nachteile durch Etablierung von stabilen Zellinien zu lösen, die die AAV-Gene rep und cap in ausreichend hoher Menge exprimieren. Da Rep jedoch toxisch ist, bereitet die Bildung von stabilen Zellklonen, die funktionsfähiges Rep78 oder Rep68 exprimieren, große Schwierigkeiten.

In WO 95/06743 wird ein Verfahren zur Herstellung von rekombinantem AAV beschrieben, bei dem als Helfervirus ein Adenoviruskonstrukt verwendet wird, welches ein die rep- und cap-Gene von AAV aufweisendes rekombinantes Insert umfaßt. Die Expression von Rep-Proteinen in mit AAV infizierten Zellen hemmt jedoch die Adenovirusinfektion, so daß keine hochtitrigen Präparationen erhalten werden können. In WO 95/06743 wird auch die Verwendung eines Herpesvirusvektors anstelle des Adenovirusvektors vorgeschlagen. Allerdings wird keine nacharbeitbare Anleitung gegeben, auf welche Weise stabile rekombinante Herpesviren, die AAV-Genbereiche umfassen, hergestellt werden können, bei denen keine Reversion zum Wildtyp stattfindet.

Eine Aufgabe der Erfindung war es daher, ein System bereitzustellen, mit dem rekombinante AAV-Vektoren als hochtitrige Präprationen, wie sie für Anwendungen im klinischen Maßstab benötigt werden, hergestellt werden können. Eine weitere Aufgabe der Erfindung war es, ein Helferkonstrukt zur Vermehrung von AAV-Vektoren bereitzustellen, bei dem die Nachteile des Standes der Technik mindestens teilweise beseitigt sind.

Diese Aufgaben werden erfindungsgemäß durch stabile Zellinien gelöst, die die AAV-Gene rep und cap in ausreichend hoher Menge exprimieren, und in denen AAV-Vektoren durch Infektion propagiert werden können.

Die Erfindung betrifft ein rekombinantes Herpesvirus, welches dadurch gekennzeichnet ist, daß es ein rep- und ein cap-Gen von Adeno-assoziierten Viren (AAV), beispielsweise ein rep- und ein cap-Gen von AAV-2 oder ein damit funktionell äquivalentes Gen, in operativer Verknüpfung mit einer Expressionskontrollsequenz enthält, wobei sich das rep- und cap- Gen auf einer Insertion befindet, die im Genom das Herpesvirus an einer geeigneten Stelle integriert ist.

Das erfindungsgemäße rekombinante Herpesvirus enthält zum einen die für die effiziente Vermehrung von AAV benötigten Helferfunktionen und kann daneben die für Rep und Cap kodierenden Bereiche von AAV in ausreichender Menge exprimieren, so daß eine Vermehrung und Verpackung von AAV-Vektoren in Zellkultur bei Coinfektion mit dem rekombinanten HSV (rHSV) möglich ist.

Als Expressionskontrollsequenzen sind alle Sequenzen geeignet, die zu einer ausreichenden Expression von rep und cap in der Zielzelle führen, beispielsweise homologe AAV-Expressionskontrollsequenzen, wie etwa der AAV p5 Promotor, oder heterologer Promotoren, z.B. eukaryontische zelluläre oder virale Promotoren. Es können konstitutive oder regulierbare Expressionskontrollsequenzen verwendet werden. Das rep- und das cap- Gen können unter gemeinsamer Kontrolle einer einzigen Expressionskontrollsequenz oder jeweils unter Kontrolle einer separaten - gleichen oder verschiedenen - Expressionskontrollsequenz stehen.

Überraschenderweise wurde festgestellt, daß Herpesviren, insbesondere HSV, gegenüber einer hohen Expression der AAV-Rep-Proteine resistent sind. Durch Integration des rep- und des cap- Gens in ein Herpesvirus, weiches ohnehin als Helfervirus für die Vermehrung der Helfervirus-abhängigen AAV-Vektoren erforderlich ist, konnte ein Konstrukt erhalten werden, welches alle zur AAV-Vermehrung benötigten Funktionen aufweist und eine Herstellung der für die Vektorpropagation benötigten AAV-Proteine in großen Mengen ermöglicht. Mit einem solchen System können infektiöse AAV-Vektoren in großem Maßstab hergestellt werden, wie sie für die Gentherapie im klinischen Maßstab benötigt werden. Die Bildung von rekombinanten AAV ist somit nicht mehr, wie bei der Verwendung von Plasmiden, von Transfektionseffizienzen abhängig.

Das erfindungsgemäße Herpesvirus ist genetisch stabil und zeigt bevorzugt keine Reversion zum Wildtyp. So findet man auch nach mehreren aufeinander folgenden Verdünnungsschritten bei einer Plaque-Reinigung, z.B. nach 3, bevorzugt nach 5 und besonders bevorzugt nach 7 Verdünnungsschritten keine sichtbare Reversion zum Wildtyp, was zeigt, daß die integrierte Kassette stabil bleibt. Ein weiterer Vorteil des erfindungsgemäßen Herpesvirus ist, daß es mit einem hohen Titer kultiviert werden kann, z.B. mit einem Titer von ≥ 5%, insbesondere ≥ 10% und besonders bevorzugt ≥ 20% des Titers des entsprechenden Herpesvirus-Wildtyps, wobei der Titer dabei bevorzugt als Cell-Release-Virus (CRV)-Titer bestimmt wird.

Das erfindungsgemäße rekombinante Herpesvirus enthält weiterhin bevorzugt ein Reportergen, dessen Exprimierbarkeit in Assoziation mit der Integration des rep- und des cap-Gens steht. Das Reportergen steht in operativer Verknüpfung mit einer geeigneten Expressionskontrollsequenz, wie etwa einem SV40-Promotor oder einem anderen Promotor, z.B. einem HSV- oder AAV-Promotor. Bevorzugt ist das Reportergen ein Nichtantibiotikumresistenz-Gen, besonders bevorzugt ein für ein direkt, z. B. visuell nachweisbares Polypeptid, z.B. LacZ oder GFP (Green Fluorescence Protein) kodierendes Gen. Durch Expression des Reportergens kann die Reinheit von AAV-Präparationen, die das rekombinante Herpesvirus nicht mehr enthalten dürfen, überwacht werden. Weiterhin wird auch eine Kontrolle der Reinheit von rekombinanten Herpesviruspräparationen gegenüber Verunreinigungen mit Wildtyp-Herpesviren ermöglicht.

Grundsätzlich kann jedes Mitglied der Herpesvirusfamilie (Herpesviridae) durch Insertion eines rep- und eines cap-Gens von AAV in ein erfindungsgemäßes rekombinantes Herpesvirus überführt werden. Beispiele geeigneter Herpesviren sind das Herpes-Simplex-Virus (HSV), das Cytomegalovirus (CMV), das Pseudorabiesvirus (PRV) und das Epstein-Barrvirus (EBV). Besonders bevorzugt ist das Herpes-Simplex-Virus (HSV) und insbesondere HSV-Typ I. Günstigerweise wird ein Herpesvirus verwendet, welches eine singuläre Restriktionsstelle aufweist, z. B. die HSV-Typ I Mutante 1802 (Rixon et al., J. Gen. Virol.71 (1990), 2934-2939), welche nur eine einzige Xbal-Stelle in der U_{S}-Region an Position 143 969 (die Nummerierung der Positionen erfolgt gemäß McGeoch et al., Nucl. Acids Res. 14 (1986), 1727-1745) aufweist.

Das Herpesvirus kann das rep- und das cap-Gen von AAV in einer nichtessentiellen Region enthalten, z.B. in der U_{S}- oder/und U_{L}- Region. Bevorzugt ist auch die Verwendung replikationsdefizienter Herpesvirusmutanten. Hierzu kann man das rep- oder/und das cap-Gen in einen Bereich des Herpesvirusgenoms inserieren, der für die Replikation des Herpesvirus benötigt wird, nicht aber für die AAV-Replikation. Alternativ kann eine Herpesvirus-Mutante verwendet werden, in der schon von vorneherein das entsprechende Herpesvirus-Replikationsgen deletiert ist. In Frage kommt beispielsweise das UL9-Gen, das für die HSV-Replikation absolut essentiell, aber für die AAV-Replikation überflüssig ist (Weindler et al., J. Virol. 65 (1991), 2476-2483). Darüber hinaus ist auch das UL54-Gen geeignet, das für das Immediate Early Protein ICP27 kodiert. Auch dieses Gen wird nicht für die AAV-Replikation benötigt. Die Deletion des UL54-Gens führt zu einer Verlangsamung des Herpesvirus-Replikationszyklus, die entsprechende Mutante ist aber jedoch nicht komplett replikationsdefizient, im Gegensatz zu einer Mutation im UL9-Gen. Darüber hinaus kommen auch noch andere Herpesvirus-Gene für die Insertion in Frage, deren Mutation oder/und Deletion den gleichen Effekt hat, nämlich eine Verlangsamung bzw. eine vollständige Blockierung des Herpesvirus-Replikationszyklus.

Bevorzugt umfaßt das rekombinante Herpesvirus nicht die vollständige invertierte Repetitionsequenz (ITR) von AAV und besonders bevorzugt ist es vollständig frei von Anteilen der ITR-Sequenz von AAV.

Eine zusätzliche Verbesserung kann durch Verwendung regulierbarer Expressionskontrollsequenzen für das rep- oder/und das cap-Gen, insbesondere für das rep-Gen erzielt werden. Beispiele hierfür sind Expressionskontrollsequenzen, die durch Zugabe von Tetracyclin (Gossen und Bujard, Proc. Natl. Sci. USA (1992), 5547-5551) oder durch Zugabe von Ecdyson (No et al., Proc. Natl. Acad. Sci. USA (1996), 3346-3351) regulierbar sind. Alternativ können auch Promotoren verwendet werden, die die Expression von "early" und "late" Herpesvirusproteinen, z.B. von HSV steuern.

Ein weiterer Gegenstand der Erfindung ist ein Verfahren zur Herstellung eines rekombinanten Herpesvirus, wobei man das rep- und das cap-Gen von AAV in das Genom eines Herpesvirus stabil integriert. Dazu wird die Herpesvirus-DNA bevorzugt an einer oder mehreren gewünschten Stellen gespalten und ein das rep- und das cap-Gen enthaltendes DNA-Fragment, z.B. ein Plasmid,in die Herpesvirus-DNA ligiert. Die Spaltung wird bevorzugt mit Restriktionsenzymen, beispielsweise mit Xbal,durchgeführt. Alternativ können die rep- und cap-Gene auch durch homologe Rekombination in das Herpesvirusgenom inseriert werden. Das rep-cap-Konstrukt muß dann flankierende Sequenzen aufweisen, die mit der für die Insertion ins Herpesvirusgenom vorgesehenen DNA-Sequenz übereinstimmen oder zumindest eine hohe Homologie aufweisen.

Ein weiterer Gegenstand der Erfindung ist eine Nukleinsäure, die aus einem erfindungsgemäßen rekombinanten Herpesvirus stammt und das rep- und das cap-Gen von Adeno-assoziierten Viren (AAV) sowie die von einem Herpesvirus stammenden, zur Vermehrung von rekombinanten Adeno-assoziierten Virusvektoren benötigten Helferfunktionen jeweils in operativer Verknüpfung mit Expressionskontrollsequenzen enthält. Diese Nukleinsäure befindet sich vorzugsweise auf einem Vektor, insbesondere auf einem eukaryontischen Vektor.

Die Erfindung umfaßt weiterhin eine Viren-Zusammensetzung, die den erfindungsgemäßen rekombinanten Herpesvirus enthält. Eine solche Zusammensetzung ist insbesondere dadurch gekennzeichnet, daß sie frei von Wildtyp-Herpesvirus ist.

Unter dem Begriff Virus, wie er hierin verwendet wird, sind auch Virionen zu verstehen.

Die erfindungsgemäßen rekombinanten Herpesviren und Vektoren werden vorteilhaft zur Herstellung von hochtitrigen, infektiösen rAAV-Vektorpräparationen verwendet. Deshalb umfaßt die Erfindung ein Verfahren zur Herstellung infektiöser AAV-Vektorpräparationen, welches die Schritte umfaßt:
(a) Bereitstellen eines viralen Vektors auf der Basis von Adeno-assoziierten Viren (AAV), welcher eine heterologe DNA-Insertion umfaßt,
(b) Bereitstellen eines rekombinanten Herpesvirus, das ein rep- und ein cap-Gen von AAV in operativer Verknüpfung mit einer Expressionskontrollsequenz enthält,
(c) Einbringen des AAV-Vektors aus (a) und des rekombinanten Herpesvirus aus (b) in eine Zelle, z.B. durch Infektion, oder/und DNA-Transfektion,
(d) Vermehren des AAV-Vektors und
(e) Gewinnen einer infektiösen AAV-Vektorpräparation.

Anstelle des rekombinanten Herpesvirus (bzw. Virions) kann auch ein entsprechender Vektor, welcher das rep- und das cap-Gen von AAV sowie die zur Replikation und Verpackung von AAV notwendigen Helfer-funktionen enthält, eingesetzt werden.

Bevorzugt wird die Zelle mit dem rekombinanten AAV-Vektor transfiziert oder infiziert und anschließend mit dem rekombinanten Herpesvirus infiziert. Besonders bevorzugt werden sowohl der AAV-Vektor als auch das Herpesvirus durch Infektion in die Zelle eingebracht, da auf diese Weise das Auftreten einer unerwünschten illegitimen Rekombination weitestgehend unterdrückt werden kann. Für das erfindungsgemäße Verfahren kann ein replizierbares rekombinantes Herpesvirus verwendet werden. Bevorzugt wird jedoch ein nicht oder nur eingeschränkt replizierbares rekombinantes Herpesvirus eingesetzt, was zu einer weiteren Erhöhung der Ausbeute an AAV führt. Mit dem erfindungsgemäßen Verfahren ist es möglich, hochtitrige, infektiöse AAV Vektorpräparationen, insbesondere eingekapselte rAAV-Präparationen zu gewinnen.

Mit Hilfe des erfindungsgemäßen rekombinanten Herpesvirus (Virions) oder Vektors, die die zur AAV-Vermehrung benötigten Helferfunktionen umfassen sowie eine ausreichende Menge von Rep und Cap Proteinen bereitstellen, können AAV-Vektoren durch Infektion von eukaryontischen Zellen einschließlich verschiedener, allgemein verfügbarer Zellinien vermehrt werden. Ein weiterer Gegenstand der Erfindung ist deshalb eine Zelle, die einen erfindungsgemäßen rekombinanten Herpesvirus oder Vektor enthält. Die Zelle ist vorzugsweise eine Säugerzelle, insbesondere eine permanent kultivierbare Zelle. Beispiele sind Nagerzellen wie BHK-Zellen, z.B. BHK21. Es können aber auch andere Zellen, z.B. humane Zellen wie etwa Vero- oder HeLa-Zellen verwendet werden.

Die Zelle kann das Virus, den Vektor oder das Virion in extrachromosomaler Form in einer oder mehreren Kopien enthalten. Derartige Zellen sind beispielsweise durch Infektion erhältlich. Alternativ kann das Virus, der Vektor oder das Virion auch im Genom der Zelle integriert vorliegen. Bevorzugt wird die Zelle durch Infektion mit dem Virus erzeugt.

Darüber hinaus kann die Zelle weiterhin einen rekombinanten AAV-Vektor, insbesondere einen AAV-Vektor mit einer heterologe DNA-Insertion, der für therapeutisch wirksames Polypeptid kodiert, enthalten. Der AAV-Vektor kann extrachromosomal oder integriert im Genom der Zelle latent vorliegen. Er wird dann bei infektion mit rekombinantem Herpesvirus freigesetzt und repliziert dann wie nach einer AAV-Vektor-Infektion.

Die Infektion von Säugerzellen mit dem erfindungsgemäßen rekombinanten Herpesvirus ergab eine hohe Expression der rep- und cap-Gene, wobei die Ausbeuten einer Coinfektion mit AAV-Witdtyp und einem Herpes-Simplex Virus vergleichbar waren.

Schließlich betrifft die Erfindung noch ein verbessertes Verfahren zur Herstellung von infektiösen AAV-Vektorpräparationen, bei dem gegenüber bekannten Verfahren, bei denen der AAV-Vektor durch Transfektion in eine Wirtszelle eingebracht wurde, eine signifikante Verringerung des Auftretens unerwünschter illegitimer Rekombinationen gefunden wird. Das Verfahren umfaßt das Einbringen eines AAV-Vektors und eines erfindungsgemäßen rep/cap exprimierenden Herpesvirus, in eine Zelle, die Kultivierung der Zelle unter geeigneten Bedingungen zur Vermehrung des AAV-Vektors und das Gewinnen einer infektiösen AAV-Vektorpräparation aus der Zelle oder/und dem Kulturüberstand, wobei das Verfahren dadurch gekennzeichnet ist, daß der AAV-Vektor und das erfindungsgemäße Helfervirus beide durch Infektion in die Zelle eingebracht werden.

Die Erfindung wird durch die beigefügten Figuren und die folgenden Beispiele weiter erläutert.
- Figur 1: zeigt die Genomstruktur eines erfindungsgemäßen rHSV/AAV. Die Position 143 969 der Xbal-Stelle in HSV-1 1802 ist gemäß der Nummerierung von McGeoch et al., Nucl. Acids Res. Nr. 14 (1986), 1727-1745). Die Nummerierung des AAV-Genoms erfolgt gemäß der Genbankhinterlegungsnummer J01901.
- Figur 2: zeigt die Expression von AAV-Rep-Proteinen kurz (A) oder lange (B) nach einer Infektion von BHK-Zellen mit rHSV/AAV.
- Figur 3: zeigt die Expression von AAV-Cap-Proteinen kurz (A) oder lange (B) nach einer Infektion von BHK-Zellen rHSV/AAV.
- Figur 4: zeigt die Lokalisierung von Rep oder Cap Proteinen und assemblierten AAV Capsiden in rHSV/AAV-infizierten BHK-Zellen durch Immunofluoreszenz.
- Figur 5: zeigt das Ergebnis eines Replikationscenterassays, in dem das herkömmliche und das erfindungsgemäße Verfahren verglichen werden.
- Figur 6: zeigt das Ergebnis eines infektiösen Titerassays von rAAV/GFP in Rohlysaten.

### Beispiele

### 1. Material und Methoden

### 1.1 Kultivierung von Zellen

BHK-21-Zellen (Stoker et al., Nature, 203 (1964), 1355-1357; ECACC Nr. 8501143) wurden als Monoschicht in G-MEM (Gibco BRL Nr. 21710-025) mit 10% NCS: (Newborn Calf Serum) (Gibco BRL-Nr. 16010-084), 1 x Tryptosephosphat-Bouillon (Gibco BRL-Nr. 18060-02) und Pen/Strep (Seromed) bei 37°C, 5% CO₂ gezüchtet. Um BHK-Zellen in Rollerflaschen (Falcon 850 cm³, Nr. 3027) zu kultivieren, wurde ein Volumen von 50 ml Medium verwendet und die Flaschen wurden mit 0,8 U/min bei 37°C, 5% CO₂ gedreht. Zur Infektion von in den Rollerflaschen wachsenden BHK-21-Zellen wurde das Volumen auf 15 ml komplettes G-MEM reduziert. BHK-21-Zellen wurden bis zu 15 Passagen vor dem Beginn einer neuen Kultur kultiviert.

HeLa- und Vero-Zellen wurden in D-MEM (Gibco Nr. 21855-025) mit 10% FCS (Seromede Nr. S0115) und Pen/Strep (Seromed) gezüchtet.

### 1.2 Herstellung von infektiösen HSV und rHSV Präparationen

Wildtyp- oder rekombinante Herpes Simplex Viren (HSV) wurden durch Infektion von etwa 2 x 10⁸ BHK-21-Zellen, die in Rollerflaschen wie in Beispiel 1 beschrieben, gezüchtet wurden, in einer Multiplizität der Infektion (MOI) von 0,002 vermehrt. Der Infektionsverlauf wurde durch die Zunahme des cytopathischen Effekts (CPE) in der Zellkultur überwacht. 3 Tage nach der Infektion zeigten die meisten Zellen einen vollständigen CPE und konnten durch Schütteln im Zellkulturmedium gesammelt werden.

Nach Zentrifugation bei 1500 x g für 10 min bei 4°C wurde Virus aus zellfreiem Überstand (CRV; Cell-Released-Virus) gewonnen, in Aliqots aufgeteilt und bei -80°C eingefroren.

Das Zellpellet wurde in PBS (Phosphat-gepufferte Salzlösung) resuspendiert und für 1 min bei 4°C beschallt. Nach Zentrifugation mit 2000 x g für 10 min bei 4°C) wurde der Überstand als zellassoziiertes Virus (CAV) gewonnen, in Aliquots aufgeteilt und bei -80°C eingefroren.

### 1.3 Reinigung von HSV-Virionen

Zur Herstellung von HSV-Virionen wurden in Rollerflaschen kultivierte BHK-Zellen wie in 1.2 beschrieben infiziert. Nach Zentrifugation wurde der CRVenthaltende klare Überstand in Zentrifugationsröhrchen überführt. Virionen wurden durch Zentrifugation für 2 h bei 4°C mit 23 000 x g pelletiert, was 13 500 U/min bei Verwendung eines Beckman SW28-Rotors entspricht. Das Viruspellet wurde in 1 ml MEM ohne Phenolrot (MEM-PR) resuspendiert und durch Beschallung bei 4°C (3 x für 30 sec) homogenisiert. Die Suspension wurde auf einen linearen Ficollgradienten (5% + 15% w/v in MEM-PR) in Beckman SW28 Ultraclear Röhrchen geschichtet und für 2 h bei 4°C mit 12 000 U/min (19 000 x g) zentrifugiert.

Bei Beleuchtung war die konzentrierte Virionen-Bande in der Mitte des Röhrchens sichtbar. Oberhalb der Virionen-Bande kann eine diffuse Bande sichtbar sein, welche beschädigte Partikel enthält. Die Virionen-Bande wurde durch Punktieren des Röhrchens mit einer 21 oder 23 Gauge-Nadel gesammelt, in ein neues Beckman SW28 Ultraclear Röhrchen überführt und mit MEM-PR auf ein Endvolumen von 35 ml verdünnt. Dann wurden die Virionen durch Zentrifugation für 2 h bei 4°C mit 22 200 U/min (65 000 x g) pelletiert. Das Pellet wurde in MEM-PR resuspendiert und bei -80°C gelagert. Zur Herstellung von viraler DNA wurde das Pellet in 300 µl TE-Puffer resuspendiert und in ein 1,5 ml Röhrchen zur weiteren Verarbeitung überführt.

Alternativ wurden HSV-Virionen unter Verwendung eines CsCl-Gradienten aufgereinigt.

### 1.4. Herstellung von HSV-DNA

Dem gemäß 1.3 gewonnenen, in 300 µl TE resuspendierten Virionpellet wurden SDS (Endkonzentration von 0,2 %) und Proteinase K (Endkonzentration 300 µg/ml) zugegeben. Der Ansatz wurde für mindestens 1 h bei 37°C inkubiert. Nach Zugabe von Natriumacetat pH 9,2, auf eine Endkonzentration von 0,3 M und einer Phenol/Chloroform-Extraktion (2 x Phenol/CIA (Chloroform-Isoamylalkohol), 1 x CIA), wurde die DNA durch Zentrifugation nach Zugabe von 2 Volumina Ethanol präzipitiert. Das Pellet wurde mit 70% Ethanol gewaschen, getrocknet und in TE (Tris-EDTA-Puffer, 10 mM Tris HCl, 1 mM EDTA) resuspendiert.

### 1.5 Herstellung des Plasmids psub201lac

Das auf pCH110 (Pharmacia) basierende Plasmid pFJ3, welches ein lacZ-Gen unter der Kontrolle eines SV40 Promotors enthält (Rixon et al., J. Gen. Virol. 71 (1990) 2931-2939), wurde mit BamHl gespalten und dephosphoryliert. Das Xbal-Fragment von psub201 (Samulski et al., J. Virol. 61 (1987), 3096-3101), das die Sequenz 191-4485 des Adeno-assoziierten Virus Typ 2 (Genbank Zugangs-Nr. J019901) einschließlich der rep- und der cap-Gene und der Promotoren p5, p19 und p40 aber ohne die Sequenzen der invertierten terminalen Repetitionen (ITR) enthält, wurde in pFJ3 insertiert, um psub201 lac zu ergeben.

### 1.6 Plaqueassay

Subkonfluente BHK-21 Zellen wurden mitverschiedenen Verdünnungen von HSV CRV oder CAV Präparationen infiziert. Nach 1 h Adsorption bei 37°C wurden die Zellen mit PBS gewaschen und mit G-MEM enthaltend 0,5% Sea Plaque-Agar, 5% NCS, Pen/Strep und 1 x Tryptose-Phosphat-Bouillon überschichtet. Nach Inkubation für 3 Tage bei 37°C, 5% CO₂ wurden die Plaques gezählt und der Titer bestimmt.

### 1.7 X-Gal-Anfärbung

Infizierte oder nichtinfizierte Zellen wurden 1 x mit 150 mM NaCl, 15 mM Natriumphosphat, pH 7,3 in PBS gespült. Die Zellen wurden durch Inkubation für 5 min in kaltem PBS, enthaltend 2 % Formaldehyd und 0,2 % Glutaraldehyd fixiert. Um das Fixativ zu enfernen, wurden Zellen mit PBS gewaschen. Schließlich wurden die Zellen mit der X-Gal-Anfärbelösung enthaltend 1 mg/ml X-Gal, 5 mM Kaliumferrocyanid, 5 mM Kaliumferricyanid, 2 mM MgCl₂ in PBS überschichtet. Angefärbte Zellen wurden üblicherweise für mehrere Stunden bei 37°C inkubiert, bis eine blaue Färbung sichtbar war.

### 1.8 Westernblot

Der Westernblot wurde, wie beim Laemmli, Nature, 227 (1970) 680-685 beschrieben, durchgeführt. Die Zellen wurden 2 x mit PBS gewaschen, entweder während sie noch an die Platte anhafteten oder nachdem sie gesammelt und in einem 15 ml Röhrchen pelletiert waren. Nach Zugabe von 100 µl 1 x SDS Probenpuffer (50 mM Tris-CI, pH 6,8; 1 % β-Mercaptoethanol; 2% SDS; 0,1 % Bromphenolblau; 10% Glycerin) pro 1 x 10⁵ Zellen wurde die Suspension in ein 1,5 ml Reaktionsgefäß überführt. Die Proben wurden in kochendem Wasser für 10 min inkubiert, auf Eis gekühlt und bei -80°C gelagert. Zur Analyse wurden bis zu 30µl jeder Probe auf ein 10% SDS Polyacrylamidgel (SDS-PAGE) gegeben. Nach der Elektrophorese wurde das Gel 15 min in einer Tris-Glycinlösung (25 mM Tris-Base; 95% mM Glycin; 10% Methanol) äquilibriert. Die Proteine wurden unter Verwendung einer halbtrockenen Blotvorrichtung und 1 mA/cm² auf eine Nitrozellulosemembran (Schleicher und Schuell, BA85, Nr. 401196) transferiert. Zur Kontrolle des Transfers und der Proteinmenge wurde die Membran mit Ponceau-S (Sigma) angefärbt. Die Membran wurde in PBS, 0,3% Tween-20, 10% fettfreiem Milchpulver für 30 min bei Raumtemperatur oder über Nacht bei 4°C blockiert.

Antikörper wurden entsprechend in PBS, 0,3% Tween-20, 10% fettfreies Milchpulver verdünnt und für mindestens 1 h bei Raumtemperatur oder über Nacht bei 4°C inkubiert. Zur Rep-Detektion wurden die monoklonalen Antikörper 303.9 und 76.3 (1:10 verdünnt) verwendet (Kleinschmidt et al. Virology 206 (1995) 254-262; Wistuba et al., J. Virol. 69 (1995), 5311-5319). Zur Detektion der Cap (VP)-Proteine wurde der Antikörper B1 (1:10 verdünnt) verwendet (Wistuba et al. (1995) supra). Die Filter wurden dreimal für 10 min mit PBS, 0,3% Tween-20 bei Raumtemperatur gewaschen. Zur Detektion von AAV-Capsidstrukturen wurde der Antikörper A20 (Wistuba et al., J. Virol. 71 (1996), 1341-1352) verwendet.

Der zweite Antikörper, üblicherweise ein Anti-Maus-Antikörper-Peroxidase-Konjugat, wurde in PBS, 0,3% Tween-20, 10% fettfreiem Milchpulver verdünnt und zusammen mit dem Filter für 30 min bis 1 h bei Raumtemperatur inkubiert. Die detektierten Proteine wurden über einen ECL-Kit gemäß Angaben des Herstellers (Amersham Life Science, RPN 2106) sichtbar gemacht.

### 1.9 Immunofluoreszenz

Zellen wurden auf Deckgläsern gezüchtet und mit HSV oder rHSV in einer MOI von 1 infiziert. 24 h nach Infektion wurden die Deckgläser 3 x mit PBS (Phosphat gepufferte Salzlösung) gewaschen, 5 min in eisgekühltem Methanol inkubiert und wiederum mit PBS gewaschen. Zum Blocken wurden die Deckgläser 30 min in PBS mit 10% NCS inkubiert. Dann erfolgte eine Inkubation für 1 h in einer Feuchtkammer mit den jeweiligen Detekions-Antikörpern (siehe 1.8), welche üblicherweise 1:1 in PBS mit 10% NCS verdünnt waren. Nach dreimaligem Waschen mit PBS wurden die Deckgläser für weitere 30 min mit verdünntem Fluorescein-Isothiocyanat (FITC) markiertem Anti-Mausantikörper inkubiert. Nach drei weiteren Waschschritten mit PBS wurden die Deckgläser mit einem Fluoreszenzmikroskop analysiert.

### 1.10 Herstellung von rAAV

Für die Herstellung von rAAV Vektoren wurde das herkömmliche Verfahren (Cotransfektion) sowie das erfindungsgemäße rHSV/AAV-System verwendet. Die Experimente wurden mit BHK-21 Zellen durchgeführt, die entweder mit Wildtyp HSV Typ 1 Stamm 1802 oder mit dem erfindungsgemäßen rHSV/AAV infiziert wurden. Es wurde ein rAAV-GFP-(UF5) verwendet, der das Reporterprotein GFP (Green Fluorescence Protein) exprimiert (Zolotukhin et al., J. Virol. 70 (1996), 4646-4654). Die Plasmidpräparationen, die das rAAV Genom flankiert von den terminalen Repetitionseinheiten enthielten, wurden auf ihre integrität hinsichtlich der terminalen Repetitionen überprüft, bevor sie in den Experimenten verwendet wurden.

BHK-21 Zellen, gezüchtet auf 5,5 cm Platten, wurden für das herkömmliche Verfahren mit 10 µg rAAV-GFP (UF5) Plasmid DNA und 10 µg ΔTR DNA (rep/cap-exprimierendes Plasmid) oder für das erfindungsgemäße Verfahren nur mit 10 µg rAAV-GFP (UF5) DNA transfiziert. Nach Inkubation über Nacht bei 37°C, 5% CO₂ wurden die Zellen zweimal mit serumfreien G-MEM und einmal mit PBS gewaschen, bevor komplettes G-MEM Medium zugegeben wurde. Dann wurden die Zellen 6 bis 12 h lang entweder mit wtHSV-1 1802 in einer MOI von 1 (herkömmliches Verfahren) oder rHSV/AAV in MOIs von 0,01 bis 1 (erfindungsgemäßes Verfahren) infiziert. Nach Adsorption für eine Stunde bei 37°C wurden die Zellen einmal mit PBS gewaschen und komplettes G-MEM wurde zugegeben. Die infizierten Zellen wurden bei 37°C, 5% CO₂, bis zu einem vollständigen CPE (üblicherweise 2 bis 3 Tage) inkubiert. Zur Ernte wurden die Platten bei -80°C eingefroren und nach Auftauen wurden die Zellen in ein 15 ml Röhrchen überführt. Nach zwei weiteren Gefrierzyklen in flüssigem Stickstoff und Auftauen wurden die zellulären Trümmer durch Zentrifugation für 15 min bei 4°C mit 1500 x g entfernt. Der klare Überstand wurde als Rohlysat gesammelt und das Helfervirus wurde durch Inkubation bei 56°C für 15 bis 30 min inaktiviert. Das Lysat wurde direkt analysiert oder weiter auf einem CsCl-Gradienten gereinigt.

### 1.11 Bestimmung der Anzahl an physikalischen rAAV Partikeln

Die Anzahl an physikalischen rAAV-Partikeln wurde durch eine Dot Blot-Analyse bestimmt. Zu 5 µl der rAAV enthaltenden Probe, erhalten aus einer CsCl-Gradientenfraktion oder einem Rohlysat, wurden 20 µl 10 x DNase I-Reaktionspuffer (500 mM Tris-CI, pH 7,5; 100 mM MgCl₂; 500 µg/ml BSA), 5 µl DNase I (12 U) und 170 µl H₂O zugegeben. Nach Inkubation bei 37°C für 1 h wurden 200 µl 2 x Proteinase K-Puffer (20 mM Tris-CI, pH 8,0, 20 mM EDTA, pH 8,0, 1 % SDS) und 100 µg Proteinase K zugegeben. Nach einer weiteren Inkubation für 1 h bei 37°C wurde ein 1/10 Volumen 3 M Natriumacetat (pH 9,2) zugegeben und die Proben wurden einer Phenolextraktion (1 x Phenol, 1 Phenol/CIA, 1 x CIA) unterzogen. Nach Zugabe von 40 µg Glykogen und 2,5 Volumen 100% Ethanol wurde 30 min bei -80°C inkubiert. Schließlich wurde die DNA durch Zentrifugation für 30 min bei maximaler Geschindigkeit pelletiert. Die Pellets wurden einmal mit 70% Ethanol gewaschen, getrocknet und schließlich in 400 µl 0,4 M NaOH, 10 mM EDTA-Lösung resuspendiert.

Als Standard wurden zweifache serielle Verdünnungen von rAAV Vektor DNA (40 bis 0,3 125 ng Plasmid) in einem Volumen von 20 µl hergestellt und mit 0,4 NaOH, 10 mM EDTA-Lösung versetzt. Alle Proben wurden 5 Minuten bei 100 °C inkubiert und sofort auf Eis gekühlt. Unter Verwendung einer Dot Blot-Vorrichtung wurden die Proben auf Gene Screen-Plus Membranen (NEN Light Science Products) überführt und die DNA wurde auf den Filtern durch UV-Licht vernetzt. Die Hybridisierung wurde gemäß Church et al., supra, bei 65°C in 0,25 M Natriumphosphatpuffer, pH 7,2, 1 mM EDTA, 7 % SDS und 1 % BSA durchgeführt. Nach 30 min bis 2 h einer Vorhybridisierung wurden die Membranen über Nacht an das [α-³²P]dCTP-markierte 731 bp Notl-Fragment von rAAV-GFP hybridisiert. Die Filter wurden bei 65°C dreimal mit Waschpuffer I (20 mM Natriumphosphatpuffer, pH 7,2; 2,5% SDS; 0,25% BSA; 1 mM EDTA) und weitere dreimal mit Waschpuffer II (20 mM Natriumphosphatpuffer, pH, 7, 2; 1% SDS, 1 mM EDTA) gewaschen. Nach Exposition der Filter auf Röntgenfilme wurden die Flecken ausgeschnitten und in einem Szintillationszähler analysiert. Die Anzahl an physikalischen Partikeln wurde unter Verwendung der doppelsträngigen rAAV-Plasmid DNA als Standard berechnet.

### 1.12 Bestimmung des infektiösen Titers von rAAV-Präparationen durch einen den Replikationscenterassay

Zur Durchführung eines Replikationscenterassays wurden die Zellen geerntet, bevor rAAV aus der primär infizierten Zelle freigesetzt ist und eine Sekundärinfektion sich in der Kultur verbreitet. Etwa 5 x 10⁴ HeLa-Zellen wurden pro Vertiefung auf eine 12-Well-Platte gegeben. Nach Inkubation über Nacht wurden die Zellen entweder mit Adenovirus (MOI = 20) alleine um wtAAV nachzuweisen oder mit Adenovirus (MOI = 20) und wtAAV (MOI = 4) um rAAV nachzuweisen, infiziert. Nach 1 h Adsorption wurden die Zellen gewaschen und mit 100 µl rAAV-GFP enthaltendem Lysat infiziert. Nach 1 h wurde ein 1 ml frisches Medium zugegeben. Dann wurden die Zellen bei 37°C, 5% CO₂ für 24 h inkubiert und durch Zentrifugation pelletiert. Noch haftende Zellen wurden trypsiniert und mit den pelletierten Zellen vereinigt. Die Zellen wurden resuspendiert und unter Verwendung einer Vakuumvorrichtung auf Nitrozellulosefilter übertragen. Die Hybridisierung der Nitrozellulosefilter wurde in einer Formamidlösung (5 x SSC; 50 % Formamid; 5 x Denhardt; 50 mM Natriumphosphatpuffer, pH 7,2; 0,1 % SDS; 0,1 mg/ml Hefe tRNA) bei 42°C entweder mit dem [α-³²P]dCTP-markiertem 731 bp Notl-Fragment von rAAV-GFP oder dem [α-^{3z}P]dCTP-markierten 1465 bp-Fragment von AAV rep durchgeführt.

### 2. Ergebnisse

### 2.1 Herstellung von rekombinantem HSV/AAV

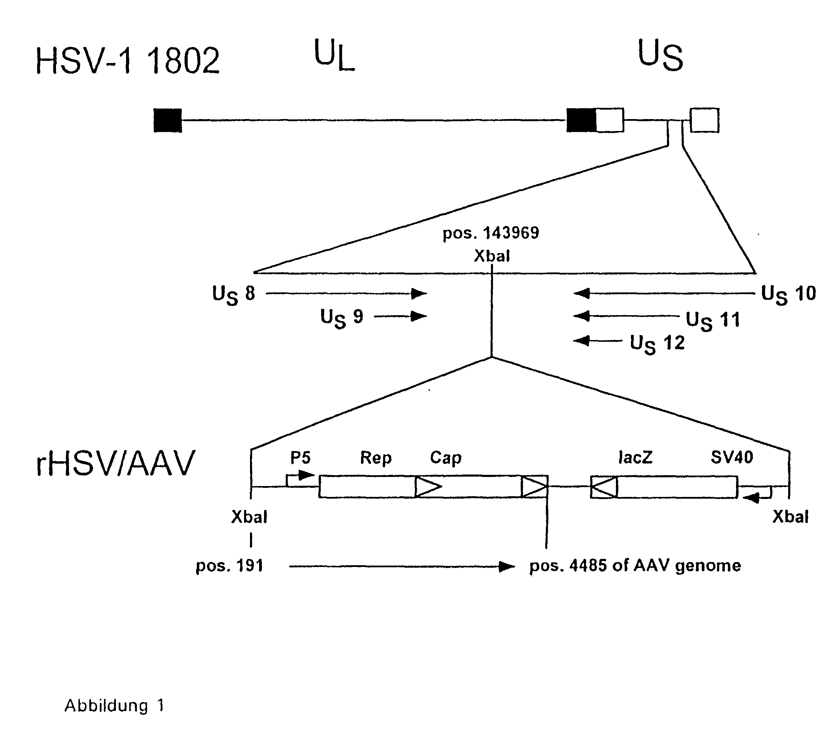

Als Ausgangsmaterial wurde die HSV-Typ I Mutante 1082 (Rixon et al., J. Gen. Virol. 71 (1990) 2931-2939) verwendet, die nur eine einzige Xbal-Stelle in der Uₛ-Region an Position 143 969 (Nummerierung gemäß McGeoch et al., supra) enthält. Diese Position ist zur Integration von heterologen Sequenzen geeignet, da zum einen keine offenen Leserahmen beeinflußt werden und zum anderen keines der 5 in diesem Bereich befindlichen Gene zur Virusvermehrung in Zellkultur essentiell ist. Die Vorgehensweise zur Herstellung von rHSV ist in Abb. 1 gezeigt.

psub201lac wurde mit Xbal gespalten, um die vollständige 8,4 kb lange Expressionskassette auszuschneiden. Anschließend wurde die gelgereinigte Expressionskassette in die Xbal-Stelle von HSV-1 1802 ligiert. Dazu wurde HSV DNA vollständig mit Xba gespalten. 1 µg der Xbal-gespaltenen HSV DNA wurde mit 1 µg gereinigtem Xbal-Fragment von psub201 lac in einem Volumen von 20 µl ligiert. Der ligierte HSV 1802/psub201 lac wurde dann in BHK-21 Zellen unter Verwendung des folgenden Verfahrens transfiziert. 1 ml HBS, 1 µl Heringsperma DNA (10 µg/µl) und 10 µg ligierter HSV 1802/psub201 lac (1 µg) wurden vermischt. Anschließend wurden 70 µl 2 M CaCl₂ tropfenweise zugegeben. Die Lösung wurde nach Entfernen des Wachstumsmediums auf BHK-Zellen gegossen. Nach Inkubation für 40 min bei 37 °C wurden 4 ml komplettes G-MEM (mit 5% NCS) zugegeben und die Zellen für weitere 200 min inkubiert. Nach Entfernen des Mediums wurden die Zellen 1 mal mit Serumfreiem G-MEM gewaschen. Die transfizierten Zellen wurden dann mit 1 ml 20 % DMSO in HBS für 4 min bei Raumtemperatur behandelt. Die DMSO-Lösung wurde entfernt und die Zellen wiederum mit serumfreiem G-MEM gewaschen. Nach Zugabe von G-MEM mit 5 % NCS wurden die Zellen 3 Tage gezüchtet, bis Plaques sichtbar waren und das Virus geerntet wurde. Die Gewinnung von CRV und CAV erfolgte wie in 1.2 beschrieben.

CAV-Plaqueassays wurden gemäß 1.6 durchgeführt. Die nach 3 Tagen sichtbaren Plaques wurden isoliert und in 200 µl PBS mit 5% NCS überführt. Nach 3 Zyklen Ein- und Auftauen wurde die Suspension verwendet um Zellen zu infizieren, die dann auf die Anwesenheit des rekombinanten Herpessimplex Virus durch β-Gal-Anfärbung und Expression der Adeno-assoziierten Virusproteine Rep oder Cap analysiert wurden. Ein positiver Plaque wurde ausgewählt und durch weitere nachfolgende Plaqueassay-Runden gereinigt. Auch wenn ein homogenes und reines rekombinantes Isolat bereits nach Runde 4 erhalten wurde, wurden weitere Plaquereinigungsrunden durchgeführt, um selbst kleinste Verunreingungen durch restlichen Wildtyp Herpes simplex Virus zu vermeiden. Alle positiven Plaques zeigten identische Muster an Rep oder Cap-Expression in der Westernblotanalyse.

Die Anwesenheit an Wildtyp HSV wurde durch Infektion verschieden großer Zellkulturschalen mit dem rekombinanten Virus und anschließendes das X-Gal Anfärben etwa 12 h p.i. untersucht. Dabei konnte keine Bildung von Wildtyp HSV angezeigt durch ungefärbte Bereiche infizierte Zellen beobachtet werden.

Das rekombinante HSV, hierin auch als rHSV/AAV bezeichnet, konnte in BHK-21 Rollerflaschen bis auf CRV-Titer von 1 bis 2 x 10⁷ PFU/ml gezüchtet werden, verglichen zu etwa 1 x 10⁸ PFU/ml, wenn Wildtyp HSV-1 1802 verwendet wurde. Die Reinheit aller rHSV/AAV-Präparationen wurde durch X-Gal-Anfärbung (siehe 1.7) analysiert. Auch bei Vermehrung von rHSV/AAV über mehrere Runden mit sehr geringen Infektionsmultiplizitäten wurde keine Reversion von rekombinantem HSV zu Wildtyp-HSV beobachtet. Unter diesen experimentellen Bedingungen würde evtl. vorhandenes Wildtyp-HSV das rHSV/AAV überwachsen, das - wie aus den obigen Titerangaben ersichtlich ist - geringfügige Wachstumsnachteile hat. Das isolierte rHSV/AAV ist dabei stabil und vermehrungsfähig. Aliquots der rHSV/AAV Präparationen wurden bei der European Collection of Cell Cultures CAMR, Salisbury, Wiltshire SP4 OIG, UK am 10. November 1997 hinterlegt und erhielten die vorläufige Zugangsnummer V97111302.

### 2.2 Expression von AAV Rep-Proteinen in mit rHSV/AAV infizierten BHK-Zellen

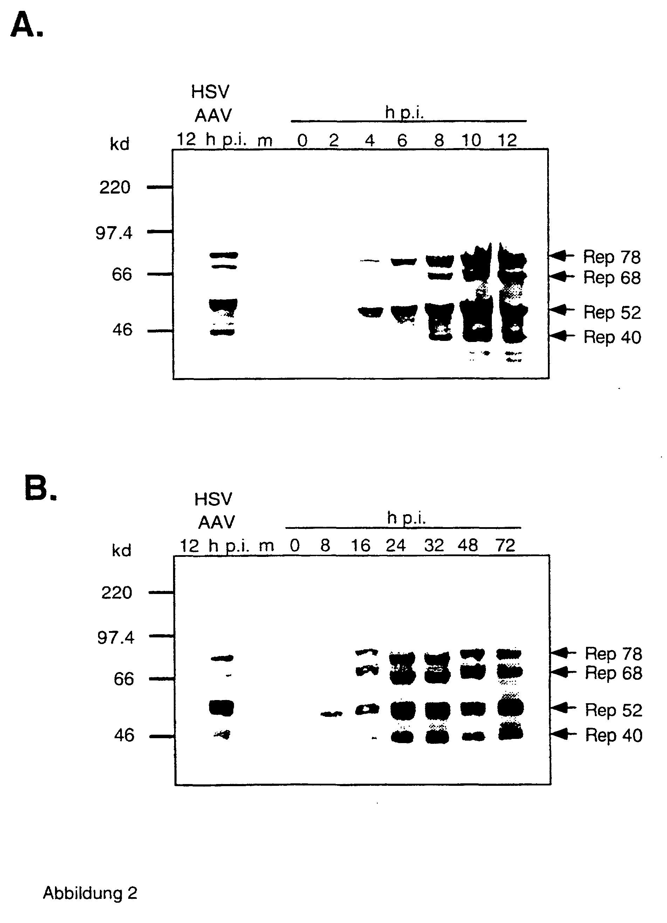

Bereits bei der Analyse während der Plaquereinigung zeigte sich, daß AAV-Proteine nach Infektion von Zellen mit rHSV/AAV exprimiert werden. Zur genaueren Analyse des Zeitverlaufs und der Expressionshöhe wurden BHK-21 Zellen mit rHSV/AAV in einer lnfektionsmultiplizität (MOI) von 1 infiziert und bei den angegebenen Zeiten nach der Infektion geerntet. AAV Rep78 und Rep52 waren in BHK-21 Zellen bereits vier Stunden nach Infektion mit rHSV/AAV nachweisbar (Abb. 2A). Nach 8 h nach Infektion (8 h p.i.) waren alle vier Rep-Proteine sichtbar, wobei die Anteile vergleichbar einer Coinfektion von AAV Wildtyp und HSV-1 1802 waren. Die Expression der Rep-Proteine war sehr hoch und lag im Bereich einer produktiven Wildtyp-AAV Infektion in Gegenwart eines Helfervirus.

Dieses Ergebnis zeigt, daß die integrierte AAV Sequenz, die die authentischen AAV Promotoren enthält, durch die HSV-spezifischen Proteine wie ein nichtintegriertes Wildtypgenom nach Coinfektion mit einem Helfervirus reguliert wird. Zusätzlich zeigen die Ergebnisse, daß ein produktiver HSV Lebenszyklus selbst durch einen sehr hohen Gehalt an Rep-Proteinen nicht signifikant inhibiert wird.

Da eine Infektion mit HSV sehr schnell den Wirtszellenmetabolismus übernimmt und schließlich die Zelle lysiert, wurde die Gegenwart von Rep-Proteinen in einem späten Stadium der Infektion untersucht. Das Ergebnis (Abb. 2B) zeigt an, daß die Expression von Rep-Proteinen zwischen 16 bis 24 h p.i. ein Plateau erreicht und Rep selbst in einem sehr späten Stadium (72 h ) nach Infektion noch nachweisbar sind.

### 2.3 Expression von Cap-Proteinen

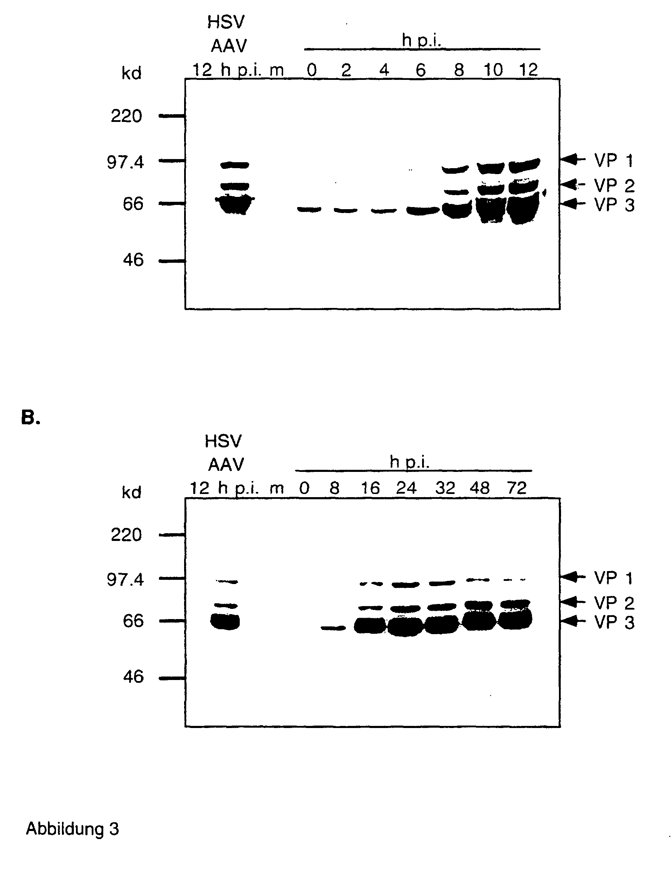

Zur effizienten Verpackung ist eine hohe Expression der Adeno-assoziierten Cap-Proteine erforderlich. Deshalb wurde der Zeitverlauf der AAV Cap-Expression in BHK-21 Zellen nach Infektion mit rHSV/AAV analysiert (Abb. 3). Das AAV VP3 Protein war bereits 0 h nach Infektion detektierbar (Abb. 3A), so daß es in das rHSV/AAV-Virion eingebaut werden kann. Zwischen 6 bis 8 h p.i. wurde eine Akkumulierung AAV VP Proteine gefunden, wobei die Mengen an VP1, VP2 und VP3 von einer Wildtyp AAV Infektion in Gegenwart von HSV-1 12 h nach Infektion nicht unterscheidbar waren (Abb. 3A). Dabei wurde eine entsprechende Expression der AAV VP Proteine erst initiiert, wenn ausreichende Gehalte an Rep-Proteinen, insbesondere Rep78 und Rep52 in den infizierten Zellen vorliegen (Abb. 2A). Die Expression der VP Proteine war von der Infektion bis zu 72 h nach Infektion hoch (Abb. 3B).

Zusammenfassend kann festgehalten werden, daß nach Infektion von BHK-21 Zellen mit rHSV/AAV hohe Mengen der VP-Proteine vorliegen, die in den Anteilen vergleichbar mit einer Coinfektion von Wildtyp AAV und HSV-1 sind.

Es wurde auch eine Expression der AAV-Proteine Cap und Rep in mit rHSV/AAV infizierten HeLa- oder Verozellen gefunden.

### 2.4 Nachweis von AAV Capsidstrukturen

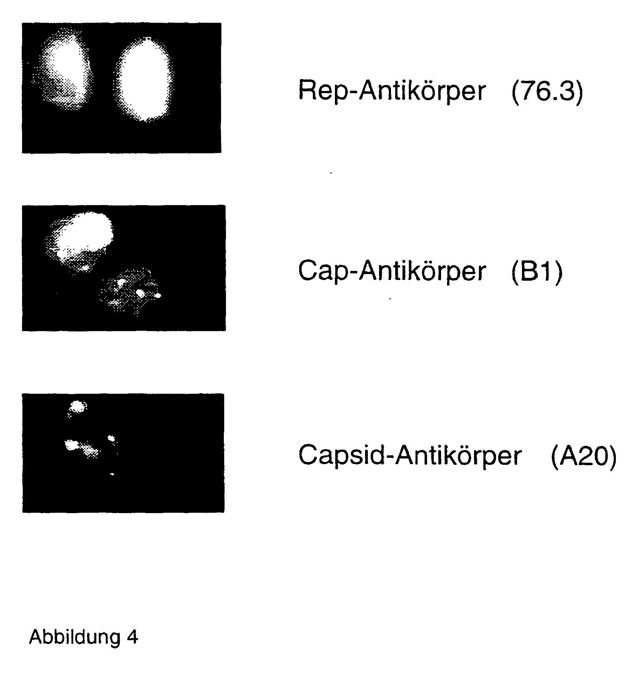

Es wurde durch Immunofluoreszenzanalyse untersucht, ob die Expression der AAV Cap-Proteine VP1, VP2 und VP3 zur Verpackung ausreicht. Unter Verwendung eines Rep-spezifischen Antikörpers (1.8) wurde eine intensive nukleäre und zytoplasmatische Anfärbung von rHSV/AAV infizierten BHK-21 Zellen beobachtet (Abb. 4). Unter Verwendung eines Cap-spezifischen Antikörpers (1.8) wurde ein ähnliches Anfärbungsmuster mit etwas geringeren Intensitäten gefunden (Abb. 4). Auch mit dem AAV-Capsidspezifischen Antikörper A20 (1.8) konnten positive Signale in rHSV/AAVinfizierten Zellen nachgewiesen werden (Abb. 4).

### 2.5 Verpackung von rekombinanten Adeno-assoziierten Virusvektoren mittels rHSV/AAV

Rekombinantes HSV/AAV unterstützt die Replikation und Verpackung von rekombinanten Adeno-assoziierten Virusvektoren, was unter Verwendung von lacZ- oder GFP-transduzierenden Adeno-assoziierten Vektoren gezeigt wurde.

Zur Bestimmung der tatsächlichen Zahl von Partikeln wurden BHK-21- oder Vero-Zellen mit rAAV-GFP (UF5) oder rAAV-GFP und ΔTR transfiziert und anschließend entweder mit rHSV/AAV oder HSV-1 1802 wie oben beschrieben, infiziert. Die Partikelzahl im Rohlysat wurde durch Dotblot bestimmt. Unter Verwendung des erfindungsgemäßen rHSV/AAV wurden 1 x 10⁴ rAAV-GFP-Partikel/Zelle gebildet verglichen mit 8 x 10³ Partikel/Zelle unter Verwendung des herkömmlichen Verfahrens.

Dazu wurden 14 cm Kulturschalen mit 100 µg UF5 oder 50 µg von jeweils UF5 und ΔTR transfiziert. Die transfizierten Zellen wurden entweder mit den rekombinanten HSV/AAV oder der HSV-1 Variante 1802 in einer MOI von 1 infiziert. Lysate wurden drei Tage p.i. hergestellt und auf die Zahl an physikalischen rAAV-GVP-Partikel analysiert. Die Membran wurde auf das [α-³²P] dCTP-markierte 731 bp Notl-Fragment von rAAV-GFP in einem Hybridisierungspuffer (Church et al. Proc. Natl. Acad. Sci. (USA) 81, (1984) 1991-1995) hybridisiert und bei 65°C inkubiert. Nach Waschen wurde der Filter freigelegt und die Flecken wurden ausgeschnitten. Die Flecken wurden in einem Packard Szintillationszähler gezählt und die Anzahl an Partikeln wurde unter Verwendung der Standards berechnet.

Die Ergebnisse sind in der folgenden Tabelle dargestellt.

**Tabelle 1**

| **Probe** | **Anzahl an Zellen** | **Anzahl rAAV Partikel** | **rAAV Partikel/Zelle** |
|---|---|---|---|
| BHK x UF5 inf. rHSV/AAV | 4 x 10⁷ | 4 x 10¹¹ | 1 x 10⁴ |
| BHK x UF5/ΔTR inf. HSV-1 | 4 x 10⁷ | 3,2 x 10¹¹ | 8 x 10³ |
| Vero x UF5 inf. rHSV/AAV | 3 x 10⁷ | 9,5 x 10¹⁰ | 3,2 x 10³ |
| Vero x UF5/ΔTR inf. HSV-1 | 3 x 10⁷ | 4 x 10" | 1,3 x 10⁴ |

### 2.5 Reinheit und Stabilität der rHSV/AAV-Präparation

Die Bildung von Wildtyp AAV (wt AAV) während einer AAV-Vektorproduktion ist nicht unüblich (siehe z.B. Muzyczka, Curr. Topics. Microbiol. Immunol. 158 (1992) 97-129) und muß deshalb beim Verpacken sorgfältig überprüft werden. Insbesondere wenn die Herstellung eines Vektors in großem Maßstab angestrebt wird, muß die Bildung von Wildtyp AAV begrenzt und kontrolliert werden. Die in rHSV/AAV integrierten AAV Sequenzen waren derart aufgebaut, daß sie frei von Sequenzelementen der invertierten terminalen Repetitionen (ITR) waren. Als Folge davon gibt es keine Sequenzüberlappungen zwischen rHSV/AAV und dem verwendeten AAV-GFP (UF5) was die Wahrscheinlichkeit eines Rekombinationsereignisses minimiert, das zur Bildung von wtAAV führen könnte. Um auszuschließen, daß wtAAV durch andere Prozesse, z.B. nichthomologe Rekombination gebildet wird, wurde ein rohes Verpackungslysat für infektiöses rAAV-GFP und wtAAV in einem Replikationscenterassay analysiert. Im Gegensatz zum Dotblot, der physikalische Partikel mißt, zeigt der Replikationscenterassay nur die infektiösen und vermehrungsfähigen Partikel.

Zwei verschiedene Konzentrationen von UF5 (10 µg oder 20 µg) oder 10 µg von jeweils UF5 und ΔTR wurden in 4 x 10⁵ HeLa-Zellen unter Verwendung der Ca-Phosphat-Methode transfiziert. Etwa 20 h nach Transfektion wurden die Zellen mit rHSV in einer MOI von 1 (für UF5 allein) oder Adenovirus mit einer MOI von 3 (für UF5/ΔTR) infiziert. Die Zellen wurden 40 h p.i. geerntet und 3 Einfrier/Auftau-Zyklen unterworfen. Nach 30 min Inkubation bei 56°C (zur Inaktivierung des Helfervirus) wurden die Zelltrümmer durch Zentrifugation pelletiert und der klare Überstand wurde gesammelt. Um den Replikationscenterassay durchzuführen wurden in 12-Well-Platten gezüchtete HeLa-Zellen nur mit Adenovirus (MOI = 20) oder mit Adenovirus (MOI = 20) und wtAAV (MOI = 4) infiziert. Anschließend wurden die Zellen mit 100 µl von jedem der zuvor hergestellten Rohlysate infiziert. Die Zellen wurden 24 h später geerntet, resuspendiert und mittels einer Vakuumvorrichtung auf Nitrozellulosemembranen überführt. Die Filter wurden mit einer gfp-spezifischen Sonde hybridisiert, um rAAV-infizierte Zellen nachzuweisen, und parallel mit einer rep-spezifischen Sonde, um Zellen sichtbar zu machen, die durch neu gebildetes wtAAV infiziert waren.

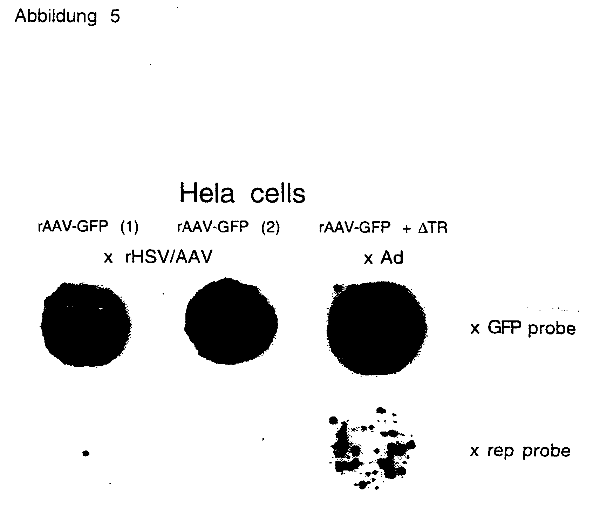

Die Ergebnisse zeigen, daß die Verwendung rHSV/AAV zusammen mit UF5 ausreichend ist, um rekombinante AAV-Vektoren zu bilden, die für eine Infektion ähnlich der herkömmlichen Cotransfektion von UF5 und ΔTR infiziert mit Adenovirus kompetent sind (Abb. 5). Die Verwendung von entweder Adenovirus- oder Adenovirus- und wtAAV-infizierten HeLa-Zellen zeigt die Bildung von Wildtyp AAV an. In diesem Experiment war die Bildung wtAAV unter Verwendung des herkömmlichen Verfahrens sehr hoch. Im Gegensatz dazu wurden durch den neuen rHSV/AAV-Ansatz praktisch keine wtAAF-Partikel gebildet, wie durch Hybridisierung an die rep-spezifische Sonde angezeigt. Dies wurde auch durch Southern Blot-Analyse bestätigt, wobei in verschiedene Verpackungspräparationen keine replikativen Formen von wtAAV gefunden wurden.

### 2.7 Herstellung infektiöser rAAV-GFP-Partiket durch Infektion mit rHSV/AAV

Es wurde der Titer an infektiösen rAAV-GFP Partikeln bestimmt. Üblicherweise ist dieser etwa 1 x 10³ niedriger als die Anzahl an physikalischen Partikeln. Rohlysate aus Ca-Phosphat-transfizierten BHK-21-Zellen wurden hierzu unter Verwendung des infektiösen Titerassays in 96-Well-Platten analysiert.

Zur Bestimmung der infektiösen Titer von wtAAV oder rAAV wurden HeLa-Zellen in 96-Well-Platten mit einem Volumen von 90 µl/Well gegeben. 10 µl der AAV enthaltenden Präparation wurden den Vertiefungen der ersten Reihe zugegen, vermischt und seriell 10fach für jeden der folgenden sieben Schritte verdünnt. Nach Inkubation für 12 bis 24 h wurden die transfizierten Zellen mit Adenovirus (MOI = 10-20) alleine infiziert, um auf wtAAV zu analysieren oder mit Adenovirus (MOI = 10-20) und wtAAV (MOI = 4), wenn der rAAv-Titer bestimmt wird. Wenn die Zellen ein vollständiges CPE zeigten, wurden sie dreimal eingefroren und aufgetaut. Die infektiösen Zellysate wurden auf eine Gene Screen® Membran übertragen. Nach Denaturieren der Membran für 5 min auf einer feuchten Schicht von Whatman-Papier, getränkt in 0,5 M NaOH, 1.5 M NaCl und Neutratisieren durch Inkubation in 20 X SSC/0,5 M Tris, pH 7,5 für 5 min wurde die DNA auf der Membran UV-vernetzt. Die Membranen wurden zur Hybridisierung mit der entsprechenden Sonde gemäß Church et al., supra, verwendet und es wurde der rAAV oder wtAAV Titer berechnet.

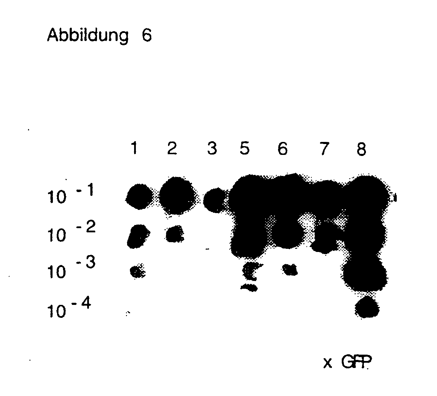

Die Ergebnisse sind in Abb. 6 dargestellt und in Tabelle 2 zusammengefaßt.

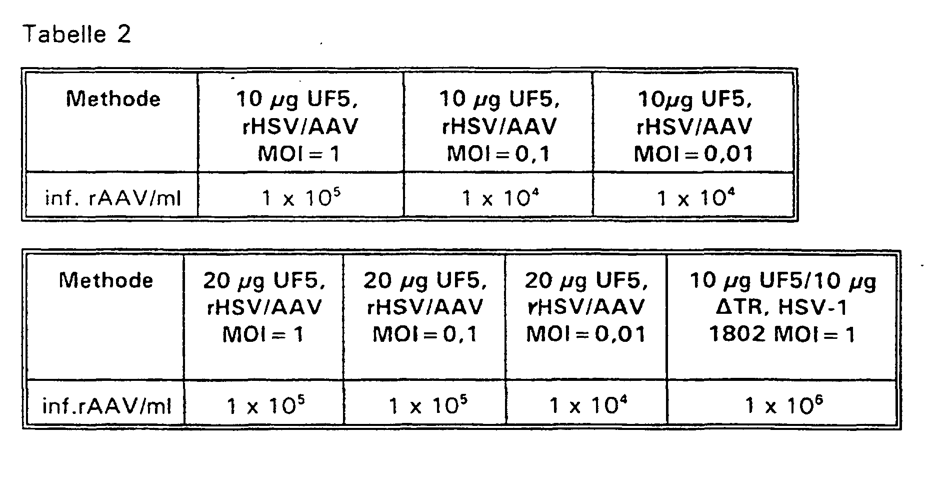

## Patentansprüche

1. Rekombinantes Herpesvirus,
**dadurch gekennzeichnet,**
**dass** es ein rep- und ein cap-Gen von Adeno-assoziierten Viren (AAV) in operativer Verknüpfung mit einer Expressionskontrollsequenz enthält, wobei sich das rep- und das cap-Gen auf einer Insertion befinden, die im Genom des Herpesvirus integriert ist.

2. Rekombinantes Herpesvirus nach Anspruch 1,
**dadurch gekennzeichnet,**
**dass** es ausgewählt ist aus der Gruppe der Herpesviridae umfassend Herpes-Simplex- Virus (HSV), Cytomegalovirus (CMV), Pseudorabiesvirus (PRV) und Epstein-Barr-Virus (EBV) und andere Mitglieder der Herpesvirusfamilie.

3. Rekombinantes Herpesvirus nach Anspruch 1 oder 2,
**dadurch gekennzeichnet,**
**dass** es sich um eine vollständig oder teilweise replikationsdefiziente Mutante handelt.

4. Verfahren zur Herstellung eines rekombinanten Herpesvirus nach einem der Ansprüche 1 bis 3,
**dadurch gekennzeichnet,**
**dass** man das rep- und das cap-Gen von AAV in das Genom eines Herpesvirus integriert.

5. Nukleinsäure umfassend die zur Replikation von AAV-Viren erforderlichen Helferfunktionen eines Herpesvirusgenoms und darin insertiert ein rep- und ein cap-Gen von Adeno-assoziierten Viren (AAV) jeweils in operativer Verknüpfung einer Expressionskontrollsequenz, wobei sich das rep- und das cap-Gen auf einer Insertion befinden, die im Genom des Herpesvirus integriert ist.

6. Vektor,
**dadurch gekennzeichnet,**
**dass** er eine Nukleinsäure nach Anspruch 5 umfasst.

7. Virale Zusammensetzung umfassend ein rekombinantes Herpesvirus nach einem der Ansprüche 1 bis 3.

8. Verfahren zur Herstellung von infektiösen AAV-Vektorpräparationen umfassen die Schritte:
(a) Bereitstellen eines viralen Vektors auf Basis von Adeno-assoziierten Viren (AAV),
(b) Bereitstellen eines rekombinanten Herpesvirus nach einem der Ansprüche 1 bis 3,
(c) Einbringen eines AAV-Vektors von (a) und des rekombinanten Herpesvirus von (b) in eine isolierte Zelle,
(d) Vermehren des AAV-Vektors und
(e) Gewinnen einer infektiösen AAV-Vektorpräparation.

9. Verfahren nach Anspruch 8,
**dadurch gekennzeichnet,**
**dass** der AAV-Vektor und der rekombinante Herpesvirus durch Infektion in die Zelle eingebracht werden.

10. Eine isolierte Zelle,
**dadurch gekennzeichnet,**
**dass** sie ein rekombinantes Herpesvirus nach einem der Ansprüche 1 bis 3 oder einen Vektor nach Anspruch 6 enthält.

11. Zelle nach Anspruch 10,
**dadurch gekennzeichnet,**
**dass** sie weiterhin einen rekombinanten AAV-Vektor enthält.

12. Zelle nach Anspruch 11,
**dadurch gekennzeichnet,**
**dass** der AAV-Vektor eine heterologe DNA-Insertion enthält, die für ein therapeutisch wirksames Polypeptid kodiert.

13. Verfahren zur Herstellung von infektiösen AAV-Vektorpräparationen, wobei ein AAV-Vektor und ein Helfervirus in eine Zelle eingebracht werden, der AAV-Vektor vermehrt und eine infektiöse AAV-Vektorpräparation aus der Zelle oder/und dem Kulturüberstand gewonnen wird,
**dadurch gekennzeichnet,**
**dass** der AAV-Vektor und das Helfervirus durch Infektion in die Zelle eingebracht werden und das Helfervirus das rekombinante Herpesvirus nach einem der Ansprüche 1 bis 3 ist.

## Claims

1. Recombinant herpesvirus, **characterized in that** it contains a rep and a cap gene derived from adeno-associated viruses (AAVs) and operatively linked to an expression control sequence, the rep gene and the cap gene being located on an insert which is integrated in the genome of the herpes virus.

2. Recombinant herpesvirus according to Claim 1, **characterized in that** it is selected from the group of Herpesviridae comprising herpes simplex virus (HSV), cytomegalovirus (CMV), pseudorabies virus (PRV) and Epstein-Barr virus (EBV) and other members of the herpesvirus family.

3. Recombinant herpesvirus according to Claim 1 or 2, **characterized in that** it is a mutant which is completely or partially replication-deficient.

4. Process for preparing a recombinant herpesvirus according to one of Claims 1 to 3, **characterized in that** the AAV rep gene and the AAV cap gene are integrated into the genome of a herpesvirus.

5. Nucleic acid which comprises the helper functions of a herpesvirus genome which are required for replicating AAV viruses and, inserted therein, a rep gene and a cap gene derived from adeno-associated viruses (AAVs), in each case operatively linked to an expression control sequence, the rep gene and the cap gene being located on an insert which is integrated in the genome of the herpes virus.

6. Vector, **characterized in that** it comprises a nucleic acid according to Claim 5.

7. Viral composition which comprises a recombinant herpervirus according to one of Claims 1 to 3.

8. Process for preparing infectious AAV vector preparations, comprising the steps of:
a) preparing a viral vector which is based on adeno-associated viruses (AAVs),
b) preparing a recombinant herpesvirus according to one of Claims 1 to 3,
c) introducing the AAV vector from (a) and the recombinant herpesvirus from (b) into an isolated cell,
d) replicating the AAV vector, and
e) obtaining an infectious AAV vector preparation.

9. Process according to Claim 18, **characterized in that** the AAV vector and the recombinant herpesvirus are introduced into the cell by infection.

10. Cell, isolated **characterized in that** it contains a recombinant herpesvirus according to one of Claims 1 to 3 or a vector according to Claim 6.

11. Cell according to Claim 10, **characterized in that** it additionally contains a recombinant AAV vector.

12. Cell according to Claim 11, **characterized in that** the AAV vector contains a heterologous DNA insert which encodes a therapeutically active polypeptide.

13. Process for producing infectious AAV vector preparations, with an AAV vector and a helper virus being introduced into a cell, the AAV vector being replicated and an infectious AAV vector preparation being obtained from the cell and/or the culture supernatant, **characterized in that** the AAV vector and the helper virus are introduced into the cell by infection and the helper virus is the recombinant herpes virus according to one of Claims 1 to 3.

## Revendications

1. Virus de l'herpès recombinant, **caractérisé en ce qu'**il contient un gène rep et un gène cap de virus adéno-associés (AAV) en liaison fonctionnelle avec une séquence de contrôle de l'expression, le gène rep et le gène cap se trouvant sur une insertion qui est intégrée dans le génome du virus de l'herpès.

2. Virus de l'herpès recombinant selon la revendication 1, **caractérisé en ce qu'**il est choisi dans le groupe des *Herpesviridae* comprenant virus de l'herpès simplex (HSV), cytomégalovirus (CMV), virus de la pseudorage (PRV) et virus d'Epstein-Barr (EBV) et d'autres membres de la famille des virus de l'herpès.

3. Virus de l'herpès recombinant selon la revendication 1 ou 2, **caractérisé en ce qu'**il s'agit d'un mutant entièrement ou partiellement déficient pour la réplication.

4. Procédé de préparation d'un virus de l'herpès recombinant selon l'une des revendications 1 à 3, **caractérisé en ce que** l'on intègre le gène rep et le gène cap d'un AAV dans le génome d'un virus de l'herpès.

5. Acide nucléique comprenant les fonctions auxiliaires d'un génome de virus de l'herpès nécessaires à la réplication de virus AAV et, insérés dans ce génome, un gène rep et un gène cap de virus adéno-associés (AAV) chacun en liaison fonctionnelle avec une séquence de contrôle de l'expression, le gène rep et le gène cap se trouvant sur une insertion qui est intégrée dans le génome du virus de l'herpès.

6. Vecteur, **caractérisé en ce qu'**il comprend un acide nucléique selon la revendication 5.

7. Composition virale comprenant un virus de l'herpès recombinant selon l'une des revendications 1 à 3.

8. Procédé de production de préparations infectieuses de vecteurs AAV comprenant les étapes de:
(a) fournir un vecteur viral à base de virus adéno-associés (AAV),
(b) fournir un virus de l'herpès recombinant selon l'une des revendications 1 à 3,
(c) introduire un vecteur AAV de (a) et le virus de l'herpès recombinant de (b) dans une cellule isolée,
(d) multiplication du vecteur AAV et
(e) obtention d'une préparation infectieuse de vecteur AAV.

9. Procédé selon la revendication 8, **caractérisé en ce que** le vecteur AAV et le virus de l'herpès recombinant sont introduits dans la cellule par infection.

10. Cellule isolée, **caractérisée en ce qu'**elle contient un virus de l'herpès recombinant selon l'une des revendications 1 à 3 ou un vecteur selon la revendication 6.

11. Cellule selon la revendication 10, **caractérisée en ce qu'**elle contient en outre un vecteur AAV recombinant.

12. Cellule selon la revendication 11, **caractérisée en ce que** le vecteur AAV contient une insertion d'ADN hétérologue qui code pour un polypeptide à activité thérapeutique.

13. Procédé de production de préparations infectieuses de vecteurs AAV, dans lequel on introduit un vecteur AAV et un virus auxiliaire dans une cellule, on multiplie le vecteur AAV et on obtient une préparation infectieuse de vecteur AAV à partir de la cellule et/ou du surnageant de culture, **caractérisé en ce que** l'on introduit le vecteur AAV et le virus auxiliaire dans la cellule par infection et le virus auxiliaire est le virus de l'herpès recombinant selon l'une des revendications 1 à 3.
